(19)

Europäisches
Patentamt
European
Patent Office
Office européen
des brevets

(11) **EP 4 559 324 A1**

(12) **EUROPEAN PATENT APPLICATION**
published in accordance with Art. 153(4) EPC

(43) Date of publication:
**28.05.2025  Bulletin 2025/22**

(21) Application number: **23842980.7**

(22) Date of filing: **18.07.2023**

(51) International Patent Classification (IPC):
*A23L 33/10* (2016.01)     *A23L 2/52* (2006.01)
*A23L 33/17* (2016.01)     *A61K 35/74* (2015.01)
*A61K 38/46* (2006.01)     *A61P 1/04* (2006.01)
*A61P 1/16* (2006.01)     *A61P 3/04* (2006.01)
*A61P 35/00* (2006.01)     *A61P 37/04* (2006.01)
*A61P 37/08* (2006.01)     *C12N 9/16* (2006.01)
*C12N 9/20* (2006.01)

(52) Cooperative Patent Classification (CPC):
**A23L 2/52; A23L 33/10; A23L 33/17; A61K 35/74;
A61K 38/46; A61P 1/04; A61P 1/16; A61P 3/04;
A61P 35/00; A61P 37/04; A61P 37/08; C12N 9/16;
C12N 9/20**

(86) International application number:
**PCT/JP2023/026314**

(87) International publication number:
**WO 2024/019057 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **20.07.2022   JP 2022115635**

(71) Applicants:
• **Amano Enzyme Inc.
Nagoya-shi
Aichi 460-8630 (JP)**
• **Hiroshima University
Higashihiroshima-shi
Hiroshima 739-8511 (JP)**
• **Keio University
Tokyo, 108-8345 (JP)**

(72) Inventors:
• **KURODA, Manabu
Kakamigahara-shi, Gifu 509-0109 (JP)**
• **YAMAGUCHI, Shotaro
Kakamigahara-shi, Gifu 509-0109 (JP)**
• **KATO, Norihisa
Higashihiroshima-shi, Hiroshima 739-8511 (JP)**
• **KUMRUNGSEE, Thanutchaporn
Higashihiroshima-shi, Hiroshima 739-8511 (JP)**
• **YANG, Yongshou
Higashihiroshima-shi, Hiroshima 739-8511 (JP)**
• **FUKUDA, Shinji
Tsuruoka-shi, Yamagata 997-0035 (JP)**

(74) Representative: **Müller-Boré & Partner
Patentanwälte PartG mbB
Friedenheimer Brücke 21
80639 München (DE)**

(54)   **AGENT FOR IMPROVING INTESTINAL FLORA**

(57)    The purpose of the present invention is to provide an agent for improving intestinal flora, the agent being capable of improving intestinal flora by increasing the number of bacteria belonging to the genus *Bifidobacterium* and/or the genus *Lactobacillus* in the intestines. In the present invention, an enzyme-containing substance derived from fungi belonging to the genus *Penicillium* increases the number of both bacteria belonging to the genus *Bifidobacterium* and bacteria belonging to the genus *Lactobacillus* in the intestines, and is capable of exhibiting the excellent effect of improving the intestinal flora.

EP 4 559 324 A1

## Description

Technical Field

The present invention relates to an agent for improving intestinal flora capable of improving intestinal flora. The present invention relates more specifically to an agent for improving intestinal flora capable of increasing the number of intestinal bacteria of genus *Bifidobacterium* and genus *Lactobacillus.*

Background Art

In recent years, the causal relationship between the intestinal environment and various diseases has been actively studied, and it has been revealed that improvement of the intestinal environment is effective for prevention or amelioration of various diseases. In the intestine, beneficial bacteria such as bacteria of genus *Bifidobacterium* and/or bacteria of genus *Lactobacillus* are mixed with harmful bacteria such as Escherichia coli, and it is thus important to form bacterial flora in which the beneficial bacteria are dominant in order to form a healthy intestinal environment.

Probiotics, prebiotics, and the like have been developed in the past, and various substances by which beneficial bacteria become predominant to improve the intestinal flora have been proposed. It has also been reported that administration of enzymes can improve the intestinal flora.

For example, Non-Patent Literature 1 has reported that the intestinal flora can be improved in mice administered a lipase derived from *Candida rugose.* Non-Patent Literature 2 has also reported that in a pig fed with a specific enzyme blend consisting of an amylase, a protease, and a xylanase together with animal feed, the number of lactobacilli is increased and the number of *Escherichia coli* is decreased in the large intestine. Non-Patent Literature 3 has also reported that in a pig fed with a specific enzyme blend (Nopcozyme II; Diasham Resources Pte Ltd.) consisting of an amylase from *Bacillus amyloliquefaciens,* a protease from *Bacillus subtilis,* and a xylanase from *Trichoderma* together with animal feed, the number of lactobacilli is increased and the numbers of *Salmonella* and *Escherichia coli* are decreased.

However, conventionally, it has not been reported that an enzyme derived from a fungus of genus *Penicillium* can increase the number of bacteria of genus *Bifidobacterium* and bacteria of genus *Lactobacillus* in the intestine.

Citation List

Non Patent Literature

Non-Patent Literature 1: Menden et al., Biomedicine & Pharmacotherapy, 2020, 130: 110579
Non-Patent Literature 2: Yi et al., Asian Astralas. J. Anim. Sci., 2013, 26: 1181-1188
Non-Patent Literature 3: Zhang et al., J. Amin. Sci., 2014, 92: 2063-2069

Summary of Invention

Technical Problem

In recent years, in response to the increasing interest in health promotion, the development of new substances by which beneficial bacteria such as bacteria of genus *Bifidobacterium* and bacteria of genus *Lactobacillus* become predominant to improve the intestinal flora has been desired.

It is thus an object of the present invention to provide an agent for improving intestinal flora that can increase the numbers of bacteria of genus *Bifidobacterium* and bacteria of genus *Lactobacillus* in the intestine to improve the intestinal flora.

Solution to Problem

The present inventors have made extensive research to solve the foregoing problem and found that an enzyme derived from a fungus of genus *Penicillium* has an action of increasing the number of bacteria of genus *Bifidobacterium* and/or bacteria of genus *Lactobacillus* in the intestine, and a substance containing an enzyme derived from a fungus of genus *Penicillium* can be used as an agent for improving intestinal flora. The present inventors have made further research based on the findings, leading to the completion of the invention.

Specifically, the present invention provides the following items of the invention.

Item 1. An agent for improving intestinal flora comprising, as an active ingredient, a substance containing an enzyme derived from a fungus of genus *Penicillium.*

Item 2. The agent for improving intestinal flora according to item 1, in which the enzyme derived from a fungus of genus *Penicillium* is a lipase and/or a nuclease derived from the fungus of genus *Penicillium.*

Item 3. The agent for improving intestinal flora according to item 1 or 2, in which the enzyme derived from a fungus of genus *Penicillium* is a lipase derived from *Penicillium camemberti* and/or a nuclease derived from *Penicillium citrinum.*

Item 4. The agent for improving intestinal flora according to any one of items 1 to 3, which is used for increasing the number of bacteria of genus *Bifidobacterium* and/or genus *Lactobacillus* in the intestine.

Item 5. A pharmaceutical preparation for oral administration for improving intestinal flora, comprising the agent for improving intestinal flora according to any one of items 1 to 4.

Item 6. A food additive for improving intestinal flora, comprising the agent for improving intestinal flora according to any one of items 1 to 4.

Item 7. A food or beverage product for improving intestinal flora, comprising the agent for improving intestinal flora according to any one of items 1 to 4.

Item 8. A method for improving intestinal flora, comprising orally applying, to an individual in need of improvement of the intestinal flora, a substance containing an enzyme derived from a fungus of genus *Penicillium.*

Item 9. A substance containing an enzyme derived from a fungus of genus *Penicillium* for use in treatment to improve intestinal flora.

Item 10. Non-therapeutic use of a substance containing an enzyme derived from a fungus of genus *Penicillium* for improving intestinal flora.

Item 11. Use of a substance containing an enzyme derived from a fungus of genus *Penicillium* for the manufacture of an agent for improving intestinal flora.

Advantageous Effects of Invention

The present invention uses a substance containing an enzyme derived from (a fungus of genus *Penicillium,* which can increase the numbers of bacteria of genus *Bifidobacterium* and bacteria of genus *Lactobacillus* in the intestine to improve the intestinal flora, and thus is effective in, for example, forming a healthy intestinal environment, maintaining a healthy intestinal environment, and preventing or treating a disease and/or a symptom due in part to an unhealthy intestinal environment.

Description of Embodiments

An agent for improving intestinal flora of the present invention is characterized by comprising, as an active ingredient, a novel substance containing an enzyme derived from a fungus of genus *Penicillium.* Hereinafter, the agent for improving intestinal flora of the present invention will be described in detail.

[Active Ingredient]

An agent for improving intestinal flora of the present invention comprises, as an active ingredient, a substance containing an enzyme derived from a fungus of genus *Penicillium.* By selecting and using the substance containing an enzyme derived from genus *Penicillium,* the numbers of both bacteria of genus *Bifidobacterium* and bacteria of genus *Lactobacillus* in the intestine can be increased to improve the intestinal flora effectively.

In the present invention, the wording "substance containing an enzyme derived from a fungus of genus *Penicillium"* refer to a material containing an enzyme derived from a fungus of genus *Penicillium,* and includes not only a product obtained by purifying one or more enzymes derived from a fungus (fungi) of genus *Penicillium* but also a mixture of one or more enzymes derived from a fungus (fungi) of genus *Penicillium* with other proteins, excipients or the like. In addition, in the present invention, the wording "substance containing an enzyme derived from a fungus of genus *Penicillium"* also includes a purified product or a crude product of culture in which a fungus (fungi) of genus *Penicillium* is cultured to produce an enzyme(s).

A microorganism from which the enzyme used in the present invention is derived is a fungus of genus *Penicillium,* and specific examples thereof include *Penicillium camemberti (P. camemberti), Penicillium citrinum (P. citrinum), Penicillium islandicum (P. islandicum), Penicillium verrucosum (P. verrucosum), Penicillium glabrum (P. glabrum), Penicillium chrysogenum (P. crysogenum), Penicillium digitatum (P. digitatum), Penicillium dodgei (P. dodgei), Penicillium expansum (P. expansum),* or *Penicillium multicolor (P. multicolor).* Among these *Penicillium* fungi, suitable examples include *Penicillium camemberti* or *Penicillium citrinum.*

In addition, the kind of the enzyme derived from a fungus of genus *Penicillium* as used in the present invention is not particularly limited, and examples thereof include a lipase, nuclease, protease, amylase, and cellulase. Among these enzymes, lipase and nuclease are preferred from the viewpoint of more effectively increasing the number of bacteria of genus *Bifidobacterium* and bacteria of genus *Lactobacillus* in the intestine.

Among the lipases derived from a fungus of genus *Penicillium,* especially the lipase derived from *Penicillium camemberti* is excellent in the action of increasing the number of bacteria of genus *Bifidobacterium* or bacteria of genus *Lactobacillus* in the intestine, and is suitably used in the present invention.

Meanwhile, the nuclease derived from a fungus of genus *Penicillium* may be a nuclease capable of hydrolyzing either RNA or DNA, or may be a nuclease capable of hydrolyzing both RNA and DNA. In addition, the nuclease derived from a fungus of genus *Penicillium* may be either an exonuclease or an endonuclease, but is preferably an exonuclease. A suitable example of the nuclease derived from a fungus of genus *Penicillium* includes 5'-phosphodiesterase. The 5'-phosphodiesterase refers to 5'-exonuclease (EC. 3.1.4.1), which is a nuclease that hydrolyzes 5'-nucleotides sequentially from the 3'-end of nucleic acid (RNA and DNA). Among the nucleases derived from *Penicillium* fungi, in particular, the nuclease derived from *Penicillium citrinum,* especially the 5'-exonuclease derived from *Penicillium citrinum* is excellent in the action of increasing the number of bacteria of genus *Bifidobacterium* or bacteria of genus *Lactobacillus* in the intestine, and is suitably used in the present invention.

The enzyme derived from genus *Penicillium* can be produced by a known production method using, for instance, a microorganism culture or a genetic engineering technique. Since the enzyme derived from genus *Penicillium* is commercially available, a commercially available product may also be used. For example, a lipase preparation derived from *Penicillium camemberti* and a 5'-exonuclease preparation derived from *Penicillium citrinum* can be obtained from Amano Enzyme Inc.

The substance containing an enzyme derived from a fungus of genus *Penicillium* as used in the present invention may comprise one kind of enzyme derived from a fungus of genus *Penicillium* or may comprise two or more kinds of enzymes derived from a fungus (fungi) of genus *Penicillium.*

[Dose]

The dose of the agent for improving intestinal flora (substance containing an enzyme derived from a fungus of genus *Penicillium*) of the present invention may be determined appropriately according to, for example, the type of active ingredient used, the type of product in which the agent is used, the use, the expected effect, and the form of application.

For example, the amount of the substance containing an enzyme derived from a fungus of genus *Penicillium* as ingested or administered per day for an adult is 0.01 to 10 g, preferably 0.05 to 5 g, and more preferably 0.1 to 2.5 g in terms of the amount of enzyme derived from a fungus of genus *Penicillium.* As used herein, the wording "in terms of the amount of enzyme derived from a fungus of genus *Penicillium"* refers to a value obtained by converting the amount of substance containing an enzyme derived from a fungus of genus *Penicillium* into the amount of enzyme derived from a fungus of genus *Penicillium* as contained in the substance containing an enzyme derived from a fungus of genus *Penicillium.*

For example, when a substance containing lipase derived from a fungus of genus *Penicillium* is used, the daily dose for an adult human as an amount of the substance containing lipase derived from a fungus of genus *Penicillium* ingested or administered may be 10,000 to 2,000,000 U, preferably 30,000 to 1,000,000 U, and more preferably 50,000 to 600,000 U in terms of the amount of lipase derived from a fungus of genus *Penicillium.* As used herein, the wording "in terms of the amount of lipase derived from a fungus of genus *Penicillium"* refers to a value obtained by converting the amount of the substance containing lipase derived from a fungus of genus *Penicillium* into the amount of lipase derived from a fungus of genus *Penicillium* as contained in the substance containing lipase. In addition, as used herein, the lipase activity (U) is a value determined using the following method:

(Method of Measuring Lipase Activity)

Vinyl laurate is added to 2 w/v% aqueous polyvinyl alcohol solution so as to have a final concentration of 3%, and the mixture is emulsified at 14,200 to 14,800 rpm for 10 minutes while being cooled to 10°C or less. The sample is stored in a refrigerator, left for 1 hour, and then immediately used.

First, 4 mL of 0.1 mol/L McIlvaine buffer (pH 5.6) and 5 mL of vinyl laurate emulsion are each accurately weighed, and placed in a flat-bottom test tube (30 × 120 mm), shaken, and left at $40 \pm 0.5$°C for 10 to 15 minutes. Then, 1 mL of sample solution containing a sample whose activity is to be measured is accurately added, and immediately shaken.

This solution is left standing at $40 \pm 0.5$°C for precisely 30 minutes, and 10 mL of liquid obtained by mixing ethanol and acetone at a volume of 1:1 is then added and shaken.

Next, 10 mL of 0.05 mol/L aqueous sodium hydroxide solution is precisely added, 10 mL of liquid obtained by mixing ethanol and acetone at a volume of 1:1 is further added and shaken, and an excessive amount of sodium hydroxide is then titrated with 0.05 mol/L hydrochloric acid ($T_{30}$ mL) (indicator: 2 drops of phenolphthalein test liquid).

Separately, as a blank, 4 mL of 0.1 mol/L McIlvaine buffer (pH 5.6) and 5 mL of vinyl laurate emulsion are each precisely weighed, and put in a flat-bottom test tube (30 × 120 mm) and shaken, and left at $40 \pm 0.5$°C for 30 minutes or more. Then, 10 mL of liquid obtained by mixing ethanol and acetone at a volume of 1:1 is added, and 1 mL of sample solution containing a sample whose activity is to be measured is then accurately added and shaken.

Next, 10 mL of 0.05 mol/L aqueous sodium hydroxide solution is precisely added, and titration is performed in the same manner as follows ($T_0$ mL).

The titration is performed by adding 0.05 mol/L hydrochloric acid to pH 10.00 while using a pH meter. Under these conditions, the amount of enzyme that results in an increase of 1 $\mu$mol of fatty acid per minute is defined as 1 U. The following equation is used for the calculation.

[Mathematical Formula 1]

$$\text{Fat digestive power (U/g, U/mL)} = 50 \times (T_0\text{-}T_{30}) \times 1/30 \times f \times n$$

$T_0$: titer value (mL) of blank
$T_{30}$: titer value (mL) of reaction solution
50: equivalent amount of fatty acids ($\mu$mol) per mL of 0.05 mol/L hydrochloric acid (for quantification)
30: reaction time (min)
f: factor of 0.05 mol/L hydrochloric acid (for quantification)
n: dilution factor per g or mL of a sample

Meanwhile, for example, when a substance containing nuclease derived from a fungus of genus *Penicillium* is used, the daily dose for an adult human as an amount of the substance containing nuclease derived from a fungus of genus *Penicillium* ingested or administered may be 1,000 to 100,000 U, preferably 3,000 to 60,000 U, and more preferably 5,000 to 30,000 U in terms of the amount of nuclease derived from a fungus of genus *Penicillium.* As used herein, the wording "in terms of the amount of nuclease derived from a fungus of genus Penicillium" refers to a value obtained by converting the amount of the substance containing nuclease derived from a fungus of genus *Penicillium* into the amount of nuclease derived from a fungus of genus *Penicillium* as contained in the substance containing nuclease. Note that as used herein, the nuclease activity of 1 U is the amount of enzyme that releases 1 $\mu$mol of phosphoric acid per minute when acts on the AMP which is a substrate. For example, when the nuclease is a phosphodiesterase, the activity can be measured in accordance with Method 1 of phosphodiesterase activity test method as specified in Ninth Edition of Japan's Specifications and Standards for Food Additives 2018 (the Ministry of Health, Labour and Welfare the Consumer Affairs Agency Japan).

[Use]

The agent for improving intestinal flora of the present invention can increase the number of beneficial bacteria such as bifidobacteria and lactic acid bacteria in the intestine, by the effect of the substance containing an enzyme derived from genus *Penicillium,* to form a beneficial bacteria-dominated flora, and thus is used for the purpose of forming a healthy intestinal environment, maintaining a healthy intestinal environment, and the like. In particular, the agent for improving intestinal flora of the present invention has an excellent effect of increasing the numbers of both bacteria of genus *Bifidobacterium* and bacteria of genus *Lactobacillus* in the intestine, and thus can also be used as an agent for increasing the numbers of bacteria of genus *Bifidobacterium* and bacteria of genus *Lactobacillus* in the intestine.

Additionally, the agent for improving intestinal flora of the present invention can form a beneficial bacteria-dominated flora to form a healthy intestinal environment, and thus can also be used for the purpose of preventing or treating a disease and/or a symptom due in part to an unhealthy intestinal environment. Examples of the disease and/or symptom include decreased immunity, colorectal cancer, allergic disease, nonalcoholic steatohepatitis, obesity, and inflammatory bowel disease.

[Form of Use]

The agent for improving intestinal flora of the present invention is orally applied by oral ingestion or oral administration. The agent for improving intestinal flora of the present invention is orally ingested or orally administered to exert the effect of improving intestinal flora after arriving at the intestine, and thus can be blended for use with various products such as foods and beverages, pharmaceutical preparations for oral administration, animal feed, and pet foods.

When the agent for improving intestinal flora of the present invention is blended with the various products, the products may contain probiotics and/or prebiotics as required together with the agent for improving intestinal flora of the present invention.

Examples of microorganisms used as the probiotics include lactic acid bacteria, bifidobacteria, and *Bacillus*

*subtilis var natto.* Specific examples of the lactic acid bacteria include lactic acid bacteria of genus *Lactobacillus,* such as *Lactobacillus casei, Lactobacillus acidophilus,* and *Lactobacillus plantarum*; lactic acid bacteria of genus *Enterococcus,* such as *Enterococcus faecalis, Enterococcus faecium,* and *Enterococcus hirae*; and lactic acid bacteria of genus *Streptococcus,* such as *Streptococcus bovis* and *Streptococcus thermophilus.* Specific examples of the bifidobacteria include *Bifidobacterium adolescentis, Bifidobacterium longum, Bifidobacterium bifidum, Bifidobacterium pseudolongum,* and *Bifidobacterium thermophilum.* These probiotics may be used alone or in combination.

Examples of the prebiotics include xylooligosaccharides, fructooligosaccharides, soybean oligosaccharides, isomaltooligosaccharides, lactosucrose, galactooligosaccharides, and lactulose. These prebiotics may be used alone or in combination.

The dosage form of a product with which the agent for improving intestinal flora of the present invention is blended may be any of a solid form, a semi-solid form, a liquid form, and the like, and is selected appropriately according to the type and use of the product.

When the agent for improving intestinal flora of the present invention is used for foods and beverages, the substance containing an enzyme derived from genus *Penicillium* is provided as a food or a beverage that exerts the effect of improving intestinal flora, either as is or in combination with other food materials or additive ingredients to be prepared in a desired form. Examples of the food and beverage include foods for specified health uses, nutritional supplements, functional foods, and foods for patients, as well as common foods and beverages. Forms of these foods and beverages include, but are not limited to, supplements, such as tablets, granules, powders, capsules, and soft capsules; and beverages, such as energy drinks, fruit beverages, carbonated beverages, and lactic acid beverages.

When the agent for improving intestinal flora of the present invention is used for foods and beverages, the amount of the agent blended into the food and beverages may be determined appropriately according to, for example, the type of active ingredient used, and the form of the food and beverage, within a range satisfying the above-described dose.

For example, when the agent for improving intestinal flora of the present invention is provided in the form of a supplement, the amount of the substance containing an enzyme derived from genus *Penicillium* blended into the supplement may be 0.001 to 0.9 g, preferably from 0.01 to 0.5 g, and more preferably from 0.02 to 0.2 g in terms of the amount of the enzyme derived from genus *Penicillium* per g of the supplement.

More specifically, when the substance containing lipase derived from genus *Penicillium* is used as the agent for improving intestinal flora of the present invention and is provided in the form of a supplement, the amount of substance containing lipase derived from genus *Penicillium* blended into the supplement may be 3,000 to 1,000,000 U/g, preferably 10,000 to 600,000 U/g, and more preferably 15,000 to 300,000 U/g in terms of the amount of the lipase derived from genus *Penicillium.*

In addition, when the substance containing nuclease derived from genus *Penicillium* is used as the agent for improving intestinal flora of the present invention and is provided in the form of a supplement, the amount of substance containing nuclease derived from genus *Penicillium* blended into the supplement may be 300 to 50,000 U/g, preferably 1,000 to 30,000 U/g, and more preferably 1,500 to 15,000 U/g in terms of the amount of the nuclease derived from genus *Penicillium.*

For example, when the agent for improving intestinal flora of the present invention is provided in the form of a beverage, the amount of the substance containing an enzyme derived from genus *Penicillium* blended into the beverage may be 0.00001 to 0.02 g/mL, preferably 0.0001 to 0.01 g/mL, and more preferably 0.0002 to 0.005 g/mL in terms of the amount of the enzyme derived from genus *Penicillium.*

More specifically, when the substance containing lipase derived from genus *Penicillium* is used as the agent for improving intestinal flora of the present invention and is provided in the form of a beverage, the amount of the substance containing lipase derived from genus *Penicillium* blended into the beverage may be 300 to 10,000 U/mL, preferably 100 to 6,000 U/mL, and more preferably 150 to 3,000 U/mL in terms of the amount of the lipase derived from genus *Penicillium.*

In addition, when the substance containing nuclease derived from genus *Penicillium* is used as the agent for improving intestinal flora of the present invention and is provided in the form of a beverage, the amount of the substance containing nuclease derived from genus *Penicillium* blended into the beverage may be 3 to 500 U/mL, preferably 10 to 300 U/mL, and more preferably 15 to 150 U/mL in terms of the amount of the nuclease derived from genus *Penicillium.*

Further, when the agent for improving intestinal flora of the present invention is used in the field of foods and beverages, the substance containing an enzyme derived from genus *Penicillium* can be provided as a food additive for improving intestinal flora, either alone or in combination with other ingredients.

When the agent for improving intestinal flora of the present invention is used for a pharmaceutical preparation for oral administration, the agent for improving intestinal flora of the present invention is provided as a pharmaceutical preparation for oral administration that exert the effect of improving intestinal flora, either alone or in combination with, for example, other pharmacologically active ingredients, pharmaceutically acceptable bases, or additives to be prepared in a desired form. Forms of the pharmaceutical preparation specifically include, but are not limited to, preparations for oral administration, such as tablets, granules, powders, capsules, soft capsules, syrups, and liquids.

Examples of the bases and additives used for manufacturing the pharmaceutical preparation for oral administration include aqueous bases such as water and alcohols, oily bases, excipients, binders, filling agents, disintegrants, lubricants, algefacients, pH-adjusting agents, thickeners, antioxidants, sequestering agents, surfactants, emulsifiers, solubilizers, solubilizing agents, flavoring agents, and antiseptics.

When the agent for improving intestinal flora of the present invention is used as a pharmaceutical preparation for oral administration, the proportion of the agent blended into the pharmaceutical preparation for oral administration may be determined appropriately according to, for example, the type of active ingredient used and the form of the pharmaceutical preparation for oral administration, within a range satisfying the above-described dose.

For example, when the agent for improving intestinal flora of the present invention is provided in the form of a solid or semi-solid pharmaceutical preparation for oral administration, the amount of the substance containing an enzyme derived from genus *Penicillium* blended into the solid or semi-solid pharmaceutical preparation for oral administration may be 0.001 to 0.5 g, preferably from 0.01 to 0.3 g, and more preferably from 0.02 to 0.15 g in terms of the amount of the enzyme derived from genus *Penicillium* per g of the pharmaceutical preparation for oral administration.

More specifically, when the substance containing lipase derived from genus *Penicillium* is used as the agent for improving intestinal flora of the present invention and is provided in the form of a solid or semi-solid pharmaceutical preparation for oral administration, the amount of the substance containing lipase derived from genus *Penicillium* blended into the solid or semi-solid pharmaceutical preparation for oral administration may be 3,000 to 1,000,000 U/g, preferably 10,000 to 600,000 U/g, and more preferably 15,000 to 300,000 U/g in terms of the amount of the lipase derived from genus *Penicillium.*

In addition, when the substance containing nuclease derived from genus *Penicillium* is used as the agent for improving intestinal flora of the present invention and is provided in the form of a solid or semi-solid pharmaceutical preparation for oral administration, the amount of the substance containing nuclease derived from genus *Penicillium* blended into the solid or semi-solid pharmaceutical preparation for oral administration may be 300 to 50,000 U/g, preferably 1,000 to 30,000 U/g, and more preferably 1,500 to 15,000 U/g in terms of the amount of the nuclease derived from genus *Penicillium.*

More specifically, when the substance containing nuclease derived from genus *Penicillium* is used as the agent for improving intestinal flora of the present invention and is provided in the form of a liquid pharmaceutical preparation for oral administration, the amount of the substance containing nuclease derived from genus *Penicillium* blended into the liquid pharmaceutical preparation for oral administration may be 30 to 10,000 U/mL, preferably 100 to 6,000 U/mL, and more preferably 150 to 3,000 U/mL in terms of the amount of the lipase derived from genus *Penicillium.*

In addition, when the substance containing nuclease derived from genus *Penicillium* is used as the agent for improving intestinal flora of the present invention and is provided in the form of a liquid pharmaceutical preparation for oral administration, the amount of the substance containing nuclease derived from genus *Penicillium* blended into the liquid pharmaceutical preparation for oral administration may be 3 to 500 U/mL, preferably 10 to 300 U/mL, and more preferably 15 to 150 U/mL in terms of the amount of the nuclease derived from genus *Penicillium.*

When the agent for improving intestinal flora of the present invention is used for animal feed or a pet food, the agent for improving intestinal flora of the present invention is provided as animal feed or a pet food that exerts the effect of improving intestinal flora, either alone or in combination with other animal feed ingredients to be prepared in a desired form. Examples of the animal feed ingredients used for the animal feed or pet food include cereal crops, such as corn, wheat, barley, and rye; bran, such as wheat bran and rice bran; dregs, such as corn gluten meal and corn germ meal; animal-derived feed, such as skimmed milk powder, whey, fish flour, and powdered bone; yeasts, such as brewer's yeast; calcium, such as calcium phosphate and calcium carbonate; vitamins; amino acids; and saccharides.

When the agent for improving intestinal flora of the present invention is used as animal feed or a pet food, the proportion of the agent blended into the animal feed or pet food may be determined appropriately according to, for example, the form and type of the animal feed or pet food, and the type of the animal to which the agent is applied.

For example, the amount of the substance containing an enzyme derived from genus *Penicillium* blended into the animal feed or per food may be 0.00001 to 0.02 g, preferably from 0.0001 to 0.01 g, and more preferably 0.0002 to 0.005 g in terms of the amount of the enzyme derived from genus *Penicillium* per g of the feed or pet food.

More specifically, when the substance containing lipase derived from genus *Penicillium* is used as the agent for improving intestinal flora of the present invention, the amount of the substance containing lipase derived from genus *Penicillium* blended into the animal feed or pet food may be 10 to 2,000 U/g, preferably 20 to 1000 U/g, and more preferably 50 to 500 U/g in terms of the amount of the lipase derived from genus *Penicillium.*

In addition, when the substance containing nuclease derived from genus *Penicillium* is used as the agent for improving intestinal flora of the present invention, the amount of the substance containing nuclease derived from genus *Penicillium* blended into the animal feed or pet food may be 1 to 200 U/g, preferably 2 to 150 U/g, and more preferably 5 to 100 U/g in terms of the amount of the nuclease derived from genus *Penicillium.*

Examples

The present invention is described more specifically below with reference to examples, although the present invention should not be construed to be limited to these examples.

Test Example 1: Effect of enzyme derived from genus *Penicillium* on intestinal flora

Using SD rats (male, 4 weeks old, from Hiroshima Institute for Experimental Animals), influence of the ingestion of an enzyme preparation containing a lipase derived from *Penicillium camemberti* (Amano Enzyme Inc.) (lipase activity: 50 U/mg) or an enzyme preparation containing a nuclease derived from *Penicillium citrinum* (5'-phosphodiesterase) (Amano Enzyme Inc.) (nuclease activity: 7U/mg) on the intestinal flora was investigated. In the experiment, the rats were divided into two groups of a control group and a working group, with eight rats per group. The rats of each group were fed *ad libitum* for 14 days with the animal feed shown in Table 1.

[Table 1]

| | Control | Example 1 | Example 2 | Example 3 |
|---|---|---|---|---|
| Enzyme preparation containing a lipase derived from *Penicillium camemberti* | - | 0.2 | 0.4 | - |
| Enzyme preparation containing a nuclease derived from *Penicillium citrinum* | - | - | - | 0.2 |
| Beef tallow | 30.0 | 30.0 | 30.0 | 30.0 |
| Casein | 20.0 | 20.0 | 20.0 | 20.0 |
| L-Cysteine | 0.3 | 0.3 | 0.3 | 0.3 |
| Vitamin mixture# | 1.0 | 1.0 | 1.0 | 1.0 |
| Mineral mixture# | 3.5 | 3.5 | 3.5 | 3.5 |
| Cellulose | 5.0 | 5.0 | 5.0 | 5.0 |
| Sucrose | 20.0 | 20.0 | 20.0 | 20.0 |
| Corn starch | 20.2 | 20.0 | 19.8 | 20.0 |
| Total | 100.0 | 100.0 | 100.0 | 100.0 |

The unit of the content of each component in the table is "% by weight".
# refers to AIN-93 (a standard purified diet composition for mice and rats formulated by the American Institute of Nutrition (AIN)).

After 14 days of feeding with the animal feed, the body weights of the rats in each group were determined, and, after extraction of DNA from the cecal contents of the rats, the 16S rRNA gene sequencing analysis (Mishima et al., Am J Physiol Renal Physiol, 2018, 315: F824-F833) was performed to quantify numbers of bacteria of genus *Bifidobacterium* and genus *Lactobacillus* in the cecal contents.

The results are shown in Table 2. In Examples 1 to 3, the numbers of bacteria of genus *Bifidobacterium* and bacteria of genus *Lactobacillus* in the intestine were both higher than those in the control. That is, it was revealed that ingestion of each enzyme derived from a fungus of genus *Penicillium* promoted the proliferation of bacteria of genus *Bifidobacterium* and bacteria of genus *Lactobacillus* in the intestine, leading to improvement of the intestinal flora.

[Table 2]

| Average body weight and 16S rRNA sequencing analysis results | | | | |
|---|---|---|---|---|
| | Control | Example 1 | Example 2 | Example 3 |
| Average body weight (g) | 203 ± 4 | 202 ± 3 | 204 ± 2 | 198 ± 3 |
| Bacteria of genus *Bifidobacterium* (% of total bacteria) | 0.06 ± 0.06 | 6.19 ± 0.79 | 7.58 ± 2.21 | 14.27± 3.16* |

(continued)

| Average body weight and 16S rRNA sequencing analysis results | | | | |
|---|---|---|---|---|
| | Control | Example 1 | Example 2 | Example 3 |
| Bacteria of genus *Lactobacillus* (% of total bacteria) | $7.26 \pm 1.87$ | $25.15 \pm 4.59$ | $46.03 \pm 8.94*$ | $56.56 \pm 8.94*$ |
| The measurement results in the table are expressed as mean $\pm$ standard deviation (SE), and * indicates $p < 0.05$ by Student's t test. | | | | |

**Claims**

1. An agent for improving intestinal flora comprising, as an active ingredient, a substance containing an enzyme derived from a fungus of genus *Penicillium.*

2. The agent for improving intestinal flora according to claim 1, wherein the enzyme derived from a fungus of genus *Penicillium* is a lipase and/or a nuclease derived from the fungus of genus *Penicillium.*

3. The agent for improving intestinal flora according to claim 1 or 2, wherein the enzyme derived from a fungus of genus *Penicillium* is a lipase derived from *Penicillium camemberti* and/or a nuclease derived from *Penicillium citrinum.*

4. The agent for improving intestinal flora according to claim 1 or 2, which is used for increasing the number of bacteria of genus *Bifidobacterium* and/or genus *Lactobacillus* in the intestine.

5. A pharmaceutical preparation for oral administration for improving intestinal flora, comprising the agent for improving intestinal flora according to claim 1 or 2.

6. A food additive for improving intestinal flora, comprising the agent for improving intestinal flora according to claim 1 or 2.

7. A food or beverage product for improving intestinal flora, comprising the agent for improving intestinal flora according to claim 1 or 2.

8. A method for improving intestinal flora, comprising orally applying, to an individual in need of improvement of the intestinal flora, a substance containing an enzyme derived from a fungus of genus *Penicillium.*

9. A substance containing an enzyme derived from a fungus of genus *Penicillium* for use in treatment to improve intestinal flora.

10. Non-therapeutic use of a substance containing an enzyme derived from a fungus of genus *Penicillium* for improving intestinal flora.

11. Use of a substance containing an enzyme derived from a fungus of genus *Penicillium* for the manufacture of an agent for improving intestinal flora.

## INTERNATIONAL SEARCH REPORT

| International application No. |
|---|
| **PCT/JP2023/026314** |

**A. CLASSIFICATION OF SUBJECT MATTER**

*A23L 33/10*(2016.01)i; *A23L 2/52*(2006.01)i; *A23L 33/17*(2016.01)i; *A61K 35/74*(2015.01)i; *A61K 38/46*(2006.01)i; *A61P 1/04*(2006.01)i; *A61P 1/16*(2006.01)i; *A61P 3/04*(2006.01)i; *A61P 35/00*(2006.01)i; *A61P 37/04*(2006.01)i; *A61P 37/08*(2006.01)i; *C12N 9/16*(2006.01)i; *C12N 9/20*(2006.01)i

FI: A23L33/10; A23L2/00 F; A23L2/52; A23L33/17; A61K35/74 D; A61K38/46; A61P1/04; A61P1/16; A61P3/04; A61P35/00; A61P37/04; A61P37/08; C12N9/16; C12N9/20

According to International Patent Classification (IPC) or to both national classification and IPC

**B. FIELDS SEARCHED**

Minimum documentation searched (classification system followed by classification symbols)

A23L33/10; A23L2/52; A23L33/17; A61K35/74; A61K38/46; A61P1/04; A61P1/16; A61P3/04; A61P35/00; A61P37/04; A61P37/08; C12N9/16; C12N9/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 5-501657 A (GIST BROCADES NV) 02 April 1993 (1993-04-02) <br> claims, p. 2, upper right column to lower left column | 1, 4-11 |
| Y | YANG, Y. et al. Supplemental Aspergillus Lipase and Protease Preparations Display Powerful Bifidogenic Effects and Modulate the Gut Microbiota Community of Rats. Fermentation. 01 December 2021, vol. 7, no. 4, article no. 294, pp. 1-13, https://doi.org/10.3390/fermentation7040294 <br> abstract, fig. 4 | 1-11 |
| Y | 山口 庄太郎, Penicillium camembertii リパーゼ ―基礎から用途開発まで―, 科学と工業, 1996, vol. 70, no. 11, pp. 473-481 <br> p. 478, right column, last paragraph, p. 479, right column, 2nd paragraph, (YAMAGUCHI, Shotaro. Penicillium camembertii Lipase. Fundamental Research and Applications. Science and Industry.) | 1-11 |

☑ Further documents are listed in the continuation of Box C.    ☑ See patent family annex.

| | |
|---|---|
| *   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **06 September 2023** | **19 September 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2023/026314** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2016-102097 A (GEORGE & OLIVER CO LTD) 02 June 2016 (2016-06-02)<br>      claim 9, paragraphs [0024], [0031] | 1-11 |
| A | JP 5851957 B2 (GEKKEIKAN SAKE CO LTD) 03 February 2016 (2016-02-03)<br>      example C1 | 1-11 |
| P, X | YANG, Y. et al. Exogenous Penicillium camemberti Lipase Preparation Exerts Prebiotic-like Effects by Increasing Cecal Bifidobacterium and Lactobacillus Abundance in Rats. Fermentation. 27 February 2023, vol. 9, no. 227, pp. 1-14, https://doi.org/10.3390/fermentation9030227<br>      abstract, p. 14, 3rd paragraph | 1-11 |
| P, Y | WO 2023/286534 A1 (AMANO ENZYME INC) 19 January 2023 (2023-01-19)<br>      claims, each example | 1-11 |

Form PCT/ISA/210 (second sheet) (January 2015)

## INTERNATIONAL SEARCH REPORT
### Information on patent family members

International application No.

**PCT/JP2023/026314**

| Patent document cited in search report | | | Publication date (day/month/year) | Patent family member(s) | | | Publication date (day/month/year) |
|---|---|---|---|---|---|---|---|
| JP | 5-501657 | A | 02 April 1993 | WO 1992/001389 A1 claims, pp. 3-4 EP 468596 A1 | | | |
| JP | 2016-102097 | A | 02 June 2016 | (Family: none) | | | |
| JP | 5851957 | B2 | 03 February 2016 | (Family: none) | | | |
| WO | 2023/286534 | A1 | 19 January 2023 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MENDEN et al.** *Biomedicine & Pharmacotherapy*, 2020, vol. 130, 110579
- **YI et al.** *Asian Astralas. J. Anim. Sci.*, 2013, vol. 26, 1181-1188
- **ZHANG et al.** *J. Amin. Sc*, 2014, vol. 92, 2063-2069
- Ninth Edition of Japan's Specifications and Standards for Food Additives 2018. Ministry of Health